# EUROPÄISCHE PATENTANMELDUNG

(11) **EP 4 169 488 A1**
(43) Veröffentlichungstag der Anmeldung: **26.04.2023**
(21) Anmeldenummer: 22202679.1
(22) Anmeldetag: 20.10.2022
(51) Int. Cl.: A61F 2/852, A61F 2/91

(54) **STENT**

(30) Priorität: 22.10.2021 DE 102021127509
(71) Anmelder: optimed medizinische Instrumente GmbH, 76275 Ettlingen (DE)
(72) Erfinder: Wack, Thilo, 66636 Tholey (DE); Wille, Thomas, 01968 Senftenberg (DE); Schmidt Fabian, 76137 Karlsruhe (DE); Zipse, Achim, 76532 Baden-Baden (DE)
(74) Vertreter: Manitz Finsterwald Patent- und Rechtsanwaltspartnerschaft mbB

(57) **Zusammenfassung**

Die Erfindung betrifft einen Stent zur transluminalen Implantation in Hohlorgane, insbesondere in Blutgefäße, Harnleiter, Speiseröhren, Colon, Duodenum, Luftwege oder Gallenwege, mit einem zumindest im Wesentlichen röhrenförmigen Körper, welcher sich entlang einer Längsrichtung erstreckt und der von einem komprimierten Zustand mit einem ersten Querschnittsdurchmesser in einen expandierten Zustand mit einem vergrößerten zweiten Querschnittsdurchmesser überführbar ist. Der erfindungsgemäße Stent zeichnet sich dadurch aus, dass der röhrenförmige Körper einen Innenkörper und einen Außenkörper umfasst, wobei der Außenkörper den Innenkörper zumindest bereichsweise umgibt, wobei der Außenkörper zumindest um einen Abschnitt des Innenkörpers vollständig umlaufend ist, und der Außenkörper aus einem bioresorbierbaren Material geformt ist oder ein bioresorbierbares Material umfasst.

## Beschreibung

Die vorliegende Erfindung betrifft einen Stent zur transluminalen Implantation in Hohlorgane, insbesondere in Blutgefäße, Harnleiter, Speiseröhren, Kolon, Duodenum, Luftwege oder Gallenwege, mit einem zumindest im Wesentlichen röhrenförmigen Körper, welcher sich entlang einer Längsrichtung erstreckt. Der röhrenförmige Körper ist von einem komprimierten Zustand mit einem ersten Querschnittsdurchmesser in einen expandierten Zustand mit einem, im Vergleich zum ersten Querschnittsdurchmesser, vergrößerten zweiten Querschnittsdurchmesser überführbar.

Stents werden zur Behandlung von krankhaft veränderten Hohlorganen eingesetzt, beispielsweise wenn sich die Hohlorgane verengt haben (Stenose). Ein anderes Anwendungsgebiet ist die Behandlung von Aneurysmen. Zur Behandlung wird ein Stent im komprimierten Zustand über einen Einführkatheter an die zu behandelnde Stelle innerhalb des Hohlorgans eingeführt, wo der Stent durch Dilatation oder durch Selbstexpansion auf einen Durchmesser, der z.B. dem Durchmesser des gesunden Hohlorgans entspricht, expandiert wird, so dass beispielsweise eine Stützwirkung des Hohlorgans, insbesondere einer Gefäßwand, erreicht wird.

Bei Stents ist es insbesondere wünschenswert, dass die Stents eine hohe radiale Aufstellkraft bereitstellen, d.h. eine ausreichend große Stützwirkung besitzen. Zugleich ist gewünscht, dass die Stents entlang der Längsrichtung flexibel ausgebildet sind, um Bewegungen des Hohlorgans folgen zu können.

In der Praxis muss allerdings häufig ein Kompromiss zwischen radialer Aufstellkraft und Flexibilität des Stents gefunden werden.

Weiterhin ist es wichtig, dass der Stent auf einfache Weise in ein Hohlorgan eingesetzt und dort möglichst genau platziert werden kann.

Es ist daher die der Erfindung zugrundeliegende Aufgabe, einen verbesserten Stent bereitzustellen, der insbesondere den vorstehend genannten Aspekten Rechnung trägt.

Diese Aufgabe wird durch einen Stent gemäß Anspruch 1 gelöst.

Der erfindungsgemäße Stent zeichnet sich dadurch aus, dass der röhrenförmige Körper einen, insbesondere röhrenförmigen, Innenkörper und einen, insbesondere ebenfalls röhrenförmigen, Außenkörper umfasst, wobei der Außenkörper den Innenkörper zumindest bereichsweise umgibt, wobei der Außenkörper zumindest um einen Abschnitt des Innenkörpers vollständig umlaufend ist, und der Außenkörper aus einem bioresorbierbarem Material geformt ist oder ein bioresorbierbares Material umfasst.

Die Erfindung setzt dabei auf der Erkenntnis auf, dass durch den Außenkörper aus bioresorbierbarem Material beim Einsetzen des Stents Vorteile erzielt werden können und zugleich nach dem Abbau des bioresorbierbaren Materials weitere Vorteile beim dauerhaften Verbleib des Innenkörpers im Hohlorgan bestehen.

Nach dem Einsetzen des Stents in das Hohlorgan wird der Außenkörper aus dem bioresorbierbaren Material über eine gewisse Zeitdauer hinweg abgebaut, d.h. "resorbiert". Nach Ablauf der Zeitdauer verbleibt also insbesondere nur der Innenkörper im Hohlorgan. Beim Einsetzen kann sich somit der Vorteil ergeben, dass der Außenkörper das Einsetzen vereinfacht, indem der Außenkörper beispielsweise so geformt sein kann, dass er gut an einem Einführbesteck befestigt werden kann. Ebenfalls ist es beispielsweise möglich, dass durch den Außenkörper eine genauere Positionierung des Innenkörpers und damit des Stents möglich wird, wie später noch genauer beschrieben.

Gemäß der Erfindung umgibt der Außenkörper den Innenkörper zumindest bereichsweise. Dies bedeutet, dass der Außenkörper z.B. vollständig umlaufend um die Längsrichtung in zumindest manchen Bereichen des Stents ist. Dies gilt insbesondere für den expandierten und den komprimierten Zustand. Der Außenkörper kann den Innenkörper beispielsweise in dem komprimierten Zustand halten. Der Außenkörper kann beispielsweise durch einen um die Stentabschnitte verlegten Draht oder durch ein Drahtgeflecht gebildet werden oder einen solchen Draht bzw. ein Drahtgeflecht umfassen. Anstelle des Drahts kann auch ein Faden verwendet werden, welcher durch den Innenkörper gestützt wird.

Der Innenkörper kann im Gegensatz zu dem Außenkörper aus einem dauerhaft im Hohlorgan beständigen Material geformt sein, beispielsweise aus Nickel-Titan Legierungen (Nitinol), aus Kobalt-Chrom, Kobalt-Nickel oder Platin-Chrom Legierungen. Insbesondere kann der Innenkörper aus einem eine Form speichernden Material gebildet sein ("Shape Memory"), das ab einer Grenztemperatur die gespeicherte Form einnimmt.

Zur einfacheren Positionierung unter Röntgenbeobachtung kann der Stent mehrere Röntgenmarker, insbesondere aus Tantal umfassen. Die Röntgenmarker können beispielsweise an den in Längsrichtung gesehenen Enden des Innenkörpers und/oder des Außenkörpers befestigt sein. Röntgenmarker am Außenkörper können insbesondere durch Materialverdickungen des bioresobierbaren Materials gebildet sein.

Bevorzugt erstreckt sich in Längsrichtung von zumindest einem Ende des Innenkörpers zumindest ein Röntgenmarker weg, insbesondere in Form einer Öse, wobei der Röntgenmarker eine asymmetrische Form aufweist. Ein Marker kann ein Abschnitt des Innenkörpers sein, der eine erhöhte Röntgendichtigkeit aufweist, d.h. in einem Röntgenbild besonders gut sichtbar ist. Insbesondere kann es sich bei dem Marker um eine Öse handeln, die beispielsweise mit dem vorgenannten Tantal gefüllt oder bedeckt ist.

Der Stent ist röhrenförmig und kann dementsprechend im expandierten Zustand innerhalb des Innenkörpers ein leeres, durchgängiges Volumen aufweisen, durch das z.B. ein Blutfluss möglich ist. Der Stent kann über seine gesamte Länge in Längsrichtung einen im expandierten Zustand zumindest im Wesentlichen gleichbleibenden Querschnittsdurchmesser aufweisen. Im Querschnitt kann der Stent eine kreisförmige oder elliptische Gestalt besitzen. Zudem kann die Länge des Stents in der Längsrichtung größer sein (z.B. zumindest 2x, 5x, 10x oder 30x größer) als der Querschnittsdurchmesser.

Der Innenkörper besitzt bevorzugt im expandierten und im komprimierten Zustand einen kleineren Querschnittsdurchmesser als der Außenkörper.

Anstelle von einem Innenkörper und einem Außenkörper könnte auch von einer Innenstruktur und einer Außenstruktur gesprochen werden. Durch den Begriff "Körper" soll jeweils verdeutlicht werden, dass diese Strukturen (auch wenn sie z.B. in verschiedene Stentabschnitte gegliedert sind) zusammengehören und gemeinsam z.B. das Stützen des Hohlorgans bewirken können.

Vorteilhafte Weiterbildungen der Erfindung sind der Beschreibung, den Zeichnungen sowie den Unteransprüchen zu entnehmen.

Gemäß einer ersten Ausführungsform umfasst der Innenkörper mehrere, insbesondere voneinander separate, ringförmige Abschnitte auf. Die separaten ringförmigen Abschnitte können dementsprechend nicht durch Material des Innenkörpers miteinander verbunden sein. Nach der Degradation des bioresorbierbaren Materials verbleiben somit insbesondere nur die ringförmigen Abschnitte im Hohlorgan. Sind die ringförmigen Abschnitte nicht miteinander verbunden, so erlaubt der dann im Hohlorgan verbleibende Innenkörper eine maximale Flexibilität und Anpassungsfähigkeit an Bewegungen und Verformungen des Hohlorgans, wobei aber gleichzeitig eine gute Stützwirkung durch die einzelnen Ringe erzielt werden kann. Ohne den zumindest temporär vorhandenen bioresorbierbaren Außenkörper ist es nahezu unmöglich, die einzelnen ringförmigen Abschnitte jeweils einzeln und mit gleichbleibenden Abstand zueinander im Hohlorgan zu platzieren, was in der Praxis insbesondere für selbstexpandierende Strukturen schwer möglich ist. Durch die einzelnen ringförmigen Abschnitte ergibt sich nach der Resorption des Außenkörpers dann die hohe Flexibilität des verbleibenden Innenkörpers.

Durch den hierin beschriebenen Stent wird es nun gestattet, viele separate ringförmige Abschnitte zugleich in ein Hohlorgan einzusetzen, wodurch sich ein Vorteil beim Einsetzen ergibt. Trotzdem wird, nach Abbau des bioresorbierbaren Materials, eine große Stützwirkung und Flexibilität durch die dauerhaft im Körper verbleibenden Teile des Innenkörpers erzielt.

Gemäß einer weiteren Ausführungsform sind die ringförmigen Abschnitte durch den Außenkörper gehalten. Wie oben schon angedeutet, kann der Außenkörper die ringförmigen Abschnitte also beispielsweise zueinander in einem insbesondere gleichen Abstand entlang der Längsrichtung halten. Durch den gleichen Abstand der ringförmigen Abschnitte zueinander entsteht im Hohlorgan eine gleichmäßige Stützwirkung. Durch das Halten der ringförmigen Abschnitte mittels des Außenkörpers wird zudem auch verhindert, dass die ringförmigen Abschnitte beim Einsetzen ihre Relativpositionen verändern.

Bevorzugt umfasst zumindest einer der ringförmigen Abschnitte eine Reihe von Zellen, die in Umfangsrichtung des Stents aufeinander folgen und vorzugsweise einen geschlossenen, in Umfangsrichtung des Stents umlaufenden Ring bilden.

Der Ring weist senkrecht zur Längsrichtung des Stents vorzugsweise einen durch die Zellen des Rings gebildeten ringförmig geschlossenen Querschnitt auf. Durch einen solchen in Umfangsrichtung umlaufenden und bevorzugt geschlossenen Ring kann in dem Stützabschnitt eine optimale radiale Aufstellkraft und Stützwirkung des Stents bzw. des Innenkörpers geschaffen werden. Ein ringförmiger Abschnitt kann prinzipiell durch beliebige Zellen gebildet sein. Insbesondere kann ein ringförmiger Abschnitt genau eine einzige Reihe oder genau zwei oder drei miteinander verbundene Reihen von Zellen aufweisen. Durch zwei oder drei miteinander verbundene Reihen von Zellen verlängert sich der ringförmige Abschnitt in der Längsrichtung, bietet aber auch eine erhöhte Aufstellkraft.

Gemäß einer weiteren Ausführungsform sind der Innenkörper und/oder der Außenkörper jeweils ein eigenständiger Stent. Innenkörper und/oder Außenkörper könnten also als eigenständige Stents angesehen werden. Dies bedeutet, dass jeder dieser Körper alleine in ein Hohlorgan eingesetzt werden könnte und in dem Hohlorgan eine Stützwirkung erzielen würde, wenn der jeweilige Körper von dem komprimierten Zustand mit einem ersten Querschnittsdurchmesser in den expandierten Zustand mit einem vergrößerten zweiten Querschnittsdurchmesser überführt wurde. Hierbei ist von Vorteil, dass die bekannten Herstellungsverfahren für Stents separat auf den Innenkörper und/oder den Außenkörper angewendet werden können. Nach Herstellung des Innenkörpers und des Außenkörpers kann der Innenkörper dann in den Außenkörper eingeführt werden, um den vollständigen Stent zu bilden.

Gemäß einer weiteren Ausführungsform ist der Innenkörper selbstexpandierend und/oder der Außenkörper ballonexpandierbar. Würde der Innenkörper allein betrachtet, so würde sich der Innenkörper bei fehlendem Druck von außen selbständig von dem komprimierten Zustand in den expandierten Zustand entfalten. Der Außenkörper hingegen kann nicht selbstexpandierend sein und ist stattdessen z.B. ballonexpandierbar. Dies bedeutet, dass innerhalb des Außenkörpers ein Bal-Ion angebracht werden kann, wobei der Ballon dann unter Druck aufgepumpt wird, sich vergrößert und bei dieser Vergrößerung den Außenkörper von dem komprimierten Zustand in den expandierten Zustand drängt und überführt ("Ballondilatation"). Insbesondere können der Innenkörper und der Außenkörper auf den Ballon aufgebracht werden. Ist der Innenkörper selbstexpandierend und der Außenkörper ballonexpandierbar, so drückt der Innenkörper im komprimierten Zustand des gesamten Stents von innen gegen den Außenkörper und wird auf diese Weise, zumindest zum Teil, am Außenkörper befestigt, insbesondere durch Kraftschluss.

Insbesondere ist die Kraft zum Überführen des Außenkörpers vom komprimierten Zustand in den expandierten Zustand größer als die Kraft, mit welcher der Innenkörper von Innen gegen den Außenkörper drückt. Der Stent insgesamt kann dementsprechend als ballonexpandierbar angesehen werden, wobei der Innenkörper insbesondere durch den Außenkörper im komprimierten Zustand gehalten wird.

Alternativ ist es auch möglich, dass sowohl Innenkörper als auch Außenkörper selbstexpandierend oder ballonexpandierbar sind.

Gemäß einer weiteren Ausführungsform umfasst der Innenkörper eine Vielzahl von Zellen, welche von von dem Innenkörper gebildeten Umrandungselementen definiert werden. Alternativ oder zusätzlich kann der Außenkörper eine Vielzahl von Zellen umfassen, welche von von dem Außenkörper gebildeten Umrandungselementen definiert werden.

Die Umrandungselemente (z.B. sogenannten "Struts") können entstehen, indem beim Schneiden des Innen- und/oder Außenkörpers aus insbesondere röhrenförmigem Material, Material entfernt wird, wobei die Umrandungselemente oder Stege bestehen bleiben. Die Umrandungselemente sind bevorzugt fest miteinander verbunden und insbesondere einstückig. Der Innenkörper und/oder der Außenkörper umfassen dementsprechend z.B. kein geflochtenes Material.

Eine Zelle kann durch einen Verbindungsabschnitt oder mehrere Verbindungsabschnitte mit einer oder mehreren anderen Zellen verbunden sein. Eine Zelle umfasst die genannte Aussparung sowie ihre jeweiligen Umrandungselemente, wobei die Verbindungsabschnitte zu den Umrandungselementen gehören.

Gemäß einer weiteren Ausführungsform bilden Zellen des Innenkörpers und/oder des Außenkörpers ein konvexes Polygon und weisen insbesondere eine Rautenform auf. Die vorstehend genannte Form der Zellen kann sich insbesondere ergeben, wenn die Zellen auf eine Ebene gelegt oder gedrückt würden (die sogenannte Abwicklung). Bei einem konvexen Polygon weisen alle Innenwinkel jeweils einen Winkel von ≤180° auf. Solche Zellen können auch geschlossene Zellen ("closed cells") genannt werden. Durch die Form eines konvexen Polygons und insbesondere durch die Rautenform kann sich eine hohe Aufstellkraft und damit eine hohe Stützwirkung des Stents ergeben.

Insbesondere weisen die meisten oder alle Zellen die Form eines konvexen Polygons oder eine Rautenform auf.

Alternativ oder zusätzlich können Zellen des Innenkörpers und/oder des Außenkörpers auch ein konkaves Polygon bilden. Bei einem konkaven Polygon kann zumindest ein Innenwinkel einen Winkel von >180° aufweisen. Beispielsweise können zumindest ein Teil der Umrandungselemente einer jeweiligen Zelle eine Zickzack-Form aufweisen. Durch solche Zickzack-Zellen oder allgemein durch Zellen mit der Form eines konkaven Polygons kann die Flexibilität des Stents weiter gesteigert werden. Es können auch die meisten oder alle Zellen die Form eines konkaven Polygons aufweisen.

Gemäß einer weiteren Ausführungsform weisen, insbesondere die Mehrheit oder alle, Zellen des Innenkörpers und des Außenkörpers dieselbe oder eine ähnliche Form auf, wobei die Zellen des Innenkörpers und des Außenkörpers zumindest im Wesentlichen nahezu deckungsgleich aufeinanderliegen oder versetzt zueinander angeordnet sind. Von einer ähnlichen Form kann insbesondere dann gesprochen werden, wenn die Umrandungselemente zweier Zellen beim Aufeinanderliegen einen maximalen Versatz aufweisen, der 10%, 20% oder 30% der Länge der größten Ausdehnung einer Zelle in einer Raumrichtung nicht überschreitet.

Sind die Zellen des Innenkörpers und des Außenkörpers versetzt zueinander angeordnet, so können Verbindungsabschnitte der Zellen des Außenkörpers jeweils mittig zwischen zwei Verbindungsabschnitten der Zellen des Innenkörpers angeordnet sein. Auf diese Weise entsteht ein "Phasenversatz" zwischen den Zellen von Innen- und Außenkörper. Durch die versetzt angeordneten Zellen ergibt sich der Vorteil einer höheren Wandabdeckung des Hohlorgans. Dies bedeutet, die Bereiche des Hohlorgans, welche nicht durch Umrandungselemente des Stents gestützt sind, werden verkleinert.

Weiterhin ist es möglich, dass der Außenkörper eine größere Anzahl von Zellen aufweist als der Innenkörper. Der Außenkörper kann z.B. 2x, 3x oder 5x die Anzahl der Zellen des Innenkörpers aufweisen. Die Zellen des Außenkörpers können dann kleiner sein als die Zellen des Innenkörpers. Der Außenkörper kann somit ein dichtes Geflecht bilden, das den Innenkörper umgibt.

Alternativ kann der Außenkörper auch weniger Zellen aufweisen als der Innenkörper. So kann der Außenkörper z.B. weniger als 80%, 50% oder 30% der Zellen des Innenkörpers aufweisen. Die Zellen des Außenkörpers können dann größer ausgeführt sein als die Zellen des Innenkörpers.

Gemäß einer weiteren Ausführungsform umfasst der Außenkörper, insbesondere an einer Außenseite, Schienen, die sich bevorzugt entlang der Längsrichtung erstrecken. Die Schienen können ein leichteres Ausfahren des Stents aus einem Einführbesteck ermöglichen. Auf diese Weise kann wiederum das Einsetzen des Stents verbessert werden. Bevorzugt sind die Schienen umlaufend um die Längsrichtung auf der Außenseite des Außenkörpers verteilt angeordnet. Insbesondere sind die Schienen verdickt ausgebildet, so dass, z.B. nur, die Schienen Erhöhungen auf der Außenseite des Außenkörpers bilden. Nachdem die Schienen dementsprechend aus dem bioresorbierbarem Material geformt sind oder das bioresorbierbare Material umfassen, werden die Schienen im Hohlorgan in der Zeitdauer abgebaut und behindern danach nicht z.B. die Flexibilität des Stents.

Die Schienen können bevorzugt an Verbindungsabschnitten der Zellen befestigt sein. Insbesondere können die Schienen auch einstückig mit den Zellen des Außenkörpers geformt sein.

Weiterhin kann vorgesehen sein, dass der Außenkörper Haltepunkte für ein Einführbesteck umfasst. Hierzu kann der Außenkörper zumindest an einem Ende z.B. ein oder mehrere Ösen, Ringe oder dergleichen umfassen, die eine Befestigung des Einführbestecks ermöglichen.

Gemäß einer weiteren Ausführungsform drückt zumindest im expandierten Zustand der Innenkörper gegen den Außenkörper. Wie oben bereits erwähnt, kann dies insbesondere der Fall sein, wenn es sich bei dem Innenkörper um einen selbstexpandierenden Innenkörper handelt. Durch den Druck des Innenkörpers werden Innen- und Außenkörper jeweils in gleichbleibenden Relativpositionen gehalten. Auch im komprimierten Zustand kann der Innenkörper gegen den Außenkörper drücken, so dass auch im komprimierten Zustand auf diese Weise eine Befestigung des Innenkörpers am Außenkörper erfolgt.

Bevorzugt drückt der Innenkörper allseitig radial nach außen gegen den Außenkörper. Zudem kann der Innenkörper zumindest in einem Bereich von 50%, 80% oder 100% seiner Länge gegen den Außenkörper drücken. Insbesondere kann also die gesamte Außenseite des Innenkörpers gegen den Außenkörper drücken.

Gemäß einer weiteren Ausführungsform weist der Innenkörper und/oder der Außenkörper Befestigungsmittel auf, um den jeweils anderen Körper zu befestigen, wobei die Befestigungsmittel insbesondere Haken, Vorsprünge und/oder Vertiefungen umfassen. Alternativ oder zusätzlich zum vorgenannten Druck des Innenkörpers auf den Außenkörper können also auch dedizierte Befestigungsmittel vorgesehen sein. Die Befestigungsmittel können auch z.B. ein Haft- und/oder Klebemittel umfassen, welches den Innen- und den Außenkörper aneinander befestigt. Bevorzugt umfasst der Außenkörper die Befestigungsmittel, so dass die Befestigungsmittel nach und nach vom Körper bzw. Hohlorgan resorbiert werden. Durch die genannten Haken oder Vorsprünge kann der Innenkörper durch Einhaken oder allgemein durch Formschluss an dem Außenkörper befestigt werden. Insbesondere kann einer der Körper auch Vertiefungen umfassen, in welche der andere Körper eingreift oder in welchen der andere Körper anliegt, um somit eine Relativbewegung zwischen Innen- und Außenkörper zu unterbinden.

Abgesehen von den Befestigungsmitteln kann der Außenkörper vollständig außerhalb des Innenkörpers angeordnet sein. Dies bedeutet, dass sich der Außenkörper z.B. nicht in den Innenkörper hinein erstreckt.

Gemäß einer weiteren Ausführungsform ist der Innenkörper oder der Außenkörper von einem Stentgraft umgeben. Allgemein gesagt, kann es sich bei dem Innenkörper oder dem Außenkörper um einen "covered stent" handeln. Der Stentgraft ist insbesondere eine künstliche Gefäßwand, die beispielsweise bei der Behandlung von Aneurysmen eingesetzt werden kann. Bei dem hierin beschriebenen Stent kann es sich aber auch um einen "bare stent" handeln, also einen Stent ohne Stentgraft.

Insbesondere kann der Innenkörper und/oder der Außenkörper auch mit einem Wirkstoff versetzt oder beschichtet sein. Ein solcher Wirkstoff kann anti-proliferativ wirken, um ein Zuwuchern des Stents mit Gewebe zu vermeiden. Beispielsweise können als Wirkstoff Anti-proliferative der Limus-Gruppe, der Statine, der P2Y12-Antagonisten oder der Thrombinantagonisten verwendet werden.

Gemäß einer weiteren Ausführungsform umfasst das bioresorbierbare Material Zink. Bevorzugt besteht das bioresorbierbare Material aus Zink und Silber oder umfasst Zink und Silber. Insbesondere enthält das bioresorbierbare Material 90,0 bis 99,95 Massen-% Zink und 0,05 bis 10,0 Massen-% Silber. Ein solches bioresorbierbares Material kann in der Blutbahn beispielsweise innerhalb weniger Wochen vom Körper abgebaut werden.

Alternativ kann das bioresorbierbare Material auch einen polymeren Werkstoff umfassen oder aus einem solchen bestehen, beispielsweise aus Poly-Milchsäure (PLA) oder Poly-L-Milchsäure (PLLA). Das bioresorbierbare Material kann auch eine Magnesiumlegierung umfassen oder aus einer Magnesiumlegierung bestehen. Durch die oben beschriebene Verwendung von Zink oder einer Zinklegierung kann die Röntgensichtbarkeit des Stents im Vergleich zu PLA, PLLA oder Magnesiumlegierungen aber gesteigert werden.

Das bioresorbierbare Material kann auch als biodegradierbares Material bezeichnet werden.

Weiterer Gegenstand der Erfindung ist ein Stent-System mit einem Stent der hierin beschriebenen Art und einem Ballon, welcher innerhalb des Innenkörpers angeordnet ist, wobei der Ballon ausgebildet ist, den Stent von dem komprimierten Zustand in den expandierten Zustand zu überführen. Der Ballon und der Stent können mit einem Einführbesteck verbunden sein. Das Einführbesteck kann Teil des Stentsystems sein. Mittels des Einführbestecks kann der Stent mit dem darin befindlichen Ballon an seinen Einsatzort im Hohlorgan eingebracht werden, wobei der Ballon dann am Einsatzort expandiert wird und beim Expandieren zugleich den Stent in den expandierten Zustand überführt. Das Einführbesteck kann bevorzugt am Außenkörper mit dem Stent verbunden sein.

Für das erfindungsgemäße Stent-System gelten die zum erfindungsgemäßen Stent getroffenen Aussagen entsprechend. Dies gilt insbesondere hinsichtlich Vorteilen und Ausführungsformen. Zudem versteht es sich, dass sämtliche hierin genannten Ausführungsformen miteinander kombinierbar sind, sofern nicht explizit etwas Gegenteiliges angegeben ist.

Nachfolgend wird die Erfindung rein beispielhaft unter Bezugnahme auf die Zeichnungen beschrieben. Es zeigt:
**Fig. 1** schematisch einen Stent mit Innen- und Außenkörper in perspektivischer Ansicht;
**Fig. 2** schematisch den Stent von Fig. 1 in einer Ansicht in Richtung der Längsrichtung;
**Fig. 3** Zellen von Innen- und Außenkörper einer ersten Ausführungsform;
**Fig. 4** Zellen von Innen- und Außenkörper einer zweiten Ausführungsform;
**Fig. 5** Einen Außenkörper mit zusätzlichen Schienen gemäß einer dritten Ausführungsform;
**Fig. 6** Zellen von Innen- und Außenkörper einer vierten Ausführungsform.

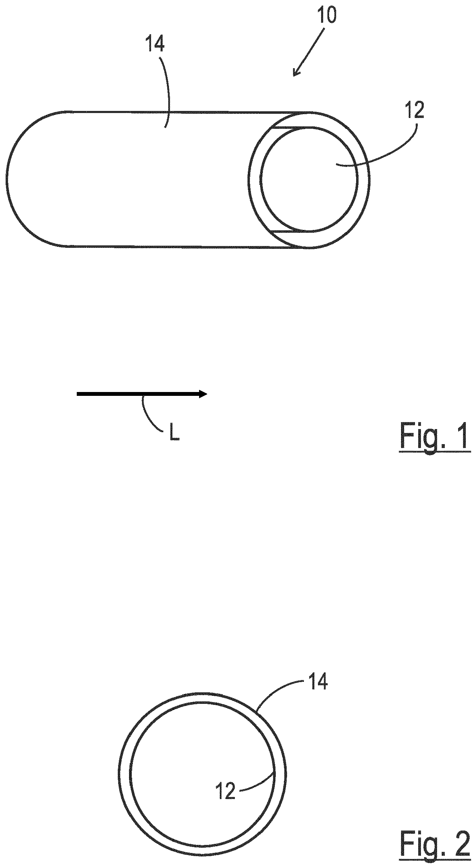

Fig. 1 zeigt einen Stent 10 im expandierten Zustand. Der Stent 10 weist eine röhrenförmige Gestalt auf und erstreckt sich entlang einer Längsrichtung L. Der Stent umfasst einen röhrenförmigen Innenkörper 12, welcher von einem ebenfalls röhrenförmigen Außenkörper 14 umgeben ist.

Der Innenkörper 12 ist aus einem dauerhaft im menschlichen Körper beständigen Material gebildet, beispielsweise Nitinol. Demgegenüber ist der Außenkörper 14 aus einem bioresorbierbaren Material geformt, beispielsweise aus einer ZinkLegierung.

Innen- und Außenkörper 12, 14 weisen zumindest im Wesentlichen dieselbe Länge entlang der Längsrichtung L auf. Der Innenkörper 12 besitzt einen kleineren Querschnittsdurchmesser als der Außenkörper 14. Der Innenkörper 12 drückt von Innen gegen den Außenkörper 14 und ist auf diese Weise durch Kraftschluss am Außenkörper 14 befestigt. Eine - alternative oder zusätzliche - formschlüssige Befestigung ist in den Figuren nicht gezeigt.

Fig. 2 zeigt den Stent von Fig. 1 in einer Ansicht in Richtung der Längsrichtung. Es ist zu erkennen, dass Innen- und Außenkörper 12, 14 im Querschnitt konzentrische Ringe bilden, d.h. dass der Außenkörper 14 den Innenkörper 12 umgibt.

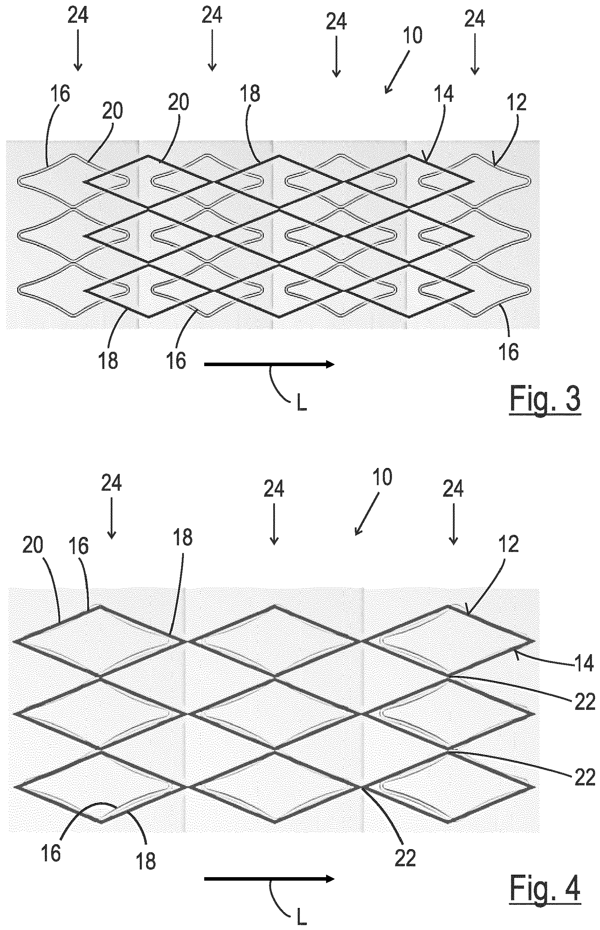

Fig. 3 zeigt nun detaillierter den Aufbau des Stents 10. Der Innenkörper 12 ist aus (Innen-)Zellen 16 gebildet. Dementsprechend ist der Außenkörper aus (Außen-) Zellen 18 geformt. Die Zellen 16, 18 weisen jeweils eine Rautenform auf und sind aus stegartigen Umrandungselementen 20 gebildet.

Fig. 3 zeigt dabei einen Ausschnitt aus einer Abwicklung des Stents 10. Dies bedeutet, der Stent 10 kann noch mehr Zellen 16, 18 umfassen, als in der Fig. 3 gezeigt. Die Innen-Zellen 16 sind jeweils über zwei Verbindungsabschnitte 22 mit zwei anderen Innen-Zellen 16 gekoppelt. Die Innen-Zellen 16 bilden so separate ringförmige Abschnitte 24, welche untereinander einen gleichen Abstand in der Längsrichtung L aufweisen.

Die Außen-Zellen 18 hingegen sind nicht in separate ringförmige Abschnitte unterteilt. Die Außen-Zellen 18 sind jeweils über drei oder vier Verbindungsabschnitte 22 untereinander verbunden, um so einen über die gesamte Länge des Außenkörpers 14 zusammenhängenden Verbund zu bilden. In der Längsrichtung gesehen, sind die Außen-Zellen 18 länger als die Innen-Zellen 16, da zwischen den Außen-Zellen 18 kein Abstand in der Längsrichtung L vorgesehen ist.

Die in Fig. 3 jeweils oben gezeigten Zellen 16, 18 sind zur Schaffung des röhrenförmigen Körpers jeweils mit den in Fig. 3 unten gezeigten Zellen verbunden.

Bei der ersten Ausführungsform von Fig. 3 sind die Innen-Zellen 16 und die Außen-Zellen 18 in der Längsrichtung versetzt zueinander angeordnet. Dies bedeutet, dass die Verbindungsabschnitte 22, welche zwei in der Längsrichtung nebeneinander angeordnete Außen-Zellen 18 verbinden, etwa mittig (z.B. über dem Diagonalenschnittpunkt) über der rautenförmigen Innen-Zelle 16 liegen.

Die in Fig. 4 gezeigte zweite Ausführungsform des Stents 10 unterscheidet sich von der ersten Ausführungsform von Fig. 3 darin, dass die Innen-Zellen 16 und die Außen-Zellen 18 nahezu deckungsgleich übereinanderliegen. Ein geringer Versatz zwischen den Zellen 16, 18 wird durch die unterschiedlichen Längen der Zellen 16, 18 verursacht. Im Übrigen gelten die Erläuterungen zur ersten Ausführungsform.

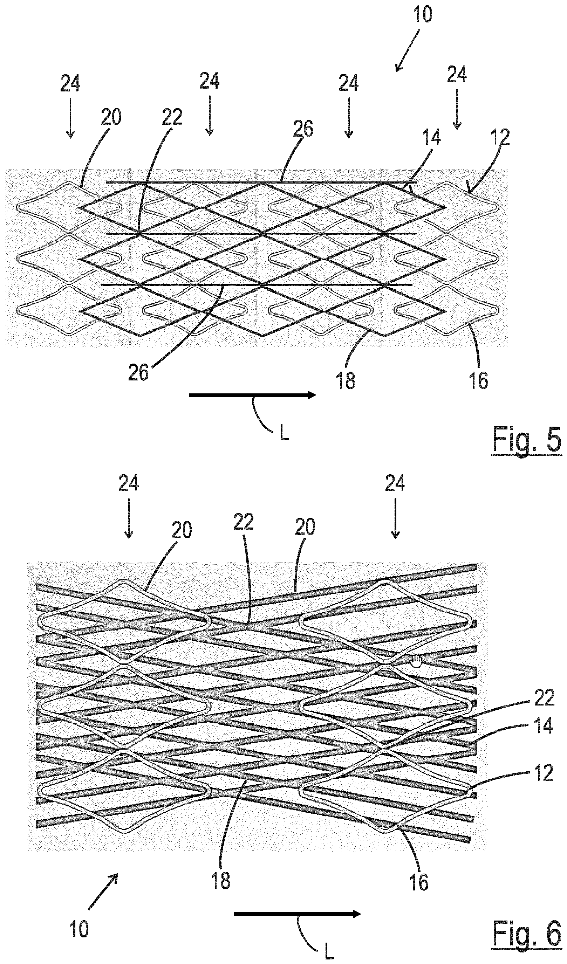

Fig. 5 zeigt eine dritte Ausführungsform des Stents 10. Die dritte Ausführungsform unterscheidet sich von der ersten Ausführungsform lediglich darin, dass der Außenkörper 14 zusätzlich in der Längsrichtung L verlaufende Schienen 26 aufweist.

Die Schienen 26 können ein Einführen des Stents 10 mit einem Einführbesteck erleichtern. Im Übrigen gelten die Erläuterungen zur ersten Ausführungsform.

Schließlich zeigt Fig. 6 eine vierte Ausführungsform des Stents 10. Bei der vierten Ausführungsform umfasst der Außenkörper 14 deutlich mehr Zellen 18 als bei der ersten Ausführungsform. Die Außen-Zellen 18 sind zudem meist kleiner als die Innen-Zellen 16. Der Außenkörper 14 umfasst dementsprechend ein dichtes Geflecht von Außen-Zellen 18. Im Übrigen gelten die Erläuterungen zur ersten Ausführungsform.

Bei sämtlichen Ausführungsformen wird der Außenkörper 14 nach einer gewissen Zeitdauer im Hohlorgan zersetzt bzw. resorbiert. Danach verbleibt nur der Innenkörper 12 im Hohlorgan. Durch die separaten ringförmigen Abschnitte 24 besitzt der so verbleibende Teil des Stents 10 eine hohe Flexibilität in der Längsrichtung L, verfügt gleichzeitig aber auch über eine hohe radiale Stützwirkung (d.h. eine hohe Kraft in Richtungen senkrecht zur Längsrichtung L).

### Bezugszeichenliste

10 Stent
12 Innenkörper
14 Außenkörper
16 Innen-Zellen
18 Außen-Zellen
20 Umrandungselement
22 Verbindungsabschnitt
24 ringförmiger Abschnitt
26 Schiene
L Längsrichtung

## Patentansprüche

1. Stent (10) zur transluminalen Implantation in Hohlorgane, insbesondere in Blutgefäße, Harnleiter, Speiseröhren, Colon, Duodenum, Luftwege oder Gallenwege, mit einem zumindest im Wesentlichen röhrenförmigen Körper, welcher sich entlang einer Längsrichtung (L) erstreckt und der von einem komprimierten Zustand mit einem ersten Querschnittsdurchmesser in einen expandierten Zustand mit einem vergrößerten zweiten Querschnittsdurchmesser überführbar ist,
**dadurch gekennzeichnet, dass**
der röhrenförmige Körper einen Innenkörper (12) und einen Außenkörper (14) umfasst, wobei der Außenkörper (14) den Innenkörper (12) zumindest bereichsweise umgibt, wobei der Außenkörper (14) zumindest um einen Abschnitt des Innenkörpers (12) vollständig umlaufend ist, und
der Außenkörper (14) aus einem bioresorbierbaren Material geformt ist oder ein bioresorbierbares Material umfasst.

2. Stent (10) nach Anspruch 1,
wobei der Innenkörper (12) mehrere, insbesondere voneinander separate, ringförmige Abschnitte (24) umfasst.

3. Stent (10) nach Anspruch 2,
wobei die ringförmigen Abschnitte (24) durch den Außenkörper (14) gehalten sind.

4. Stent (10) nach einem der vorstehenden Ansprüche,
wobei der Innenkörper (12) und/oder der Außenkörper (14) jeweils ein eigenständiger Stent sind.

5. Stent (10) nach einem der vorstehenden Ansprüche,
wobei der Innenkörper (12) selbstexpandierend und/oder der Außenkörper (14) ballonexpandierbar ist.

6. Stent (10) nach einem der vorstehenden Ansprüche,
wobei der Innenkörper (12) eine Vielzahl von Zellen (16) umfasst, welche von von dem Innenkörper (12) gebildeten Umrandungselementen (20) definiert werden,
und/oder
wobei der Außenkörper (14) eine Vielzahl von Zellen (18) umfasst, welche von von dem Außenkörper (14) gebildeten Umrandungselementen (20) definiert werden.

7. Stent (10) nach Anspruch 6,
wobei Zellen (16, 18) des Innenkörpers (12) und/oder des Außenkörpers (14) ein konvexes Polygon bilden und insbesondere eine Rautenform aufweisen.

8. Stent (10) nach Anspruch 6 oder 7,
wobei Zellen (16, 18) des Innenkörpers (12) und des Außenkörpers (14) dieselbe oder eine ähnliche Form aufweisen, wobei die Zellen (16, 18) des Innenkörpers (12) und des Außenkörpers (14) deckungsgleich aufeinander liegen oder versetzt zueinander angeordnet sind.

9. Stent (10) nach einem der vorstehenden Ansprüche,
wobei der Außenkörper (14), insbesondere an einer Außenseite, Schienen (26) umfasst, die sich entlang der Längsrichtung erstrecken.

10. Stent (10) nach einem der vorstehenden Ansprüche,
wobei zumindest im expandierten Zustand der Innenkörper (12) gegen den Außenkörper (14) drückt.

11. Stent (10) nach einem der vorstehenden Ansprüche,
wobei der Innenköper (12) und/oder der Außenkörper (14) Befestigungsmittel aufweisen, um den jeweils anderen Körper zu befestigen, wobei die Befestigungsmittel insbesondere Haken, Vorsprünge, Vertiefungen und/oder Haft- oder Klebemittel umfassen.

12. Stent (10) nach einem der vorstehenden Ansprüche,
wobei der Innenkörper (12) oder der Außenkörper (14) von einem Stentgraft umgeben ist.

13. Stent (10) nach einem der vorstehenden Ansprüche,
wobei das bioresobierbare Material Zink umfasst und bevorzugt aus Zink und Silber besteht, wobei das bioresorbierbare Material 90,0 bis 99,95 Massen-% Zink und 0,05 bis 10,0 Massen-% Silber enthält.

14. Stent-System mit einem Stent (10) gemäß zumindest einem der vorstehenden Ansprüche und einem Ballon, welcher innerhalb des Innenkörpers (12) angeordnet ist und ausgebildet ist, den Stent (10) von dem komprimierten Zustand in den expandierten Zustand zu überführen.
